# EUROPEAN PATENT SPECIFICATION

(11) **EP 4 267 014 B1**
(45) Date of publication and mention of the grant of the patent: **03.09.2025**
(21) Application number: 22703831.2
(22) Date of filing: 25.01.2022
(51) Int. Cl.: A61B 17/00, A61F 2/95, A61M 25/00

(54) **MEDICAL DELIVERY DEVICE**
MEDIZINISCHE ABGABEVORRICHTUNG
DISPOSITIF D'ADMINISTRATION MÉDICAL

(30) Priority: 19.02.2021 US 202163151092 P
(43) Date of publication of application: 01.11.2023
(73) Proprietor: Boston Scientific Scimed, Inc., Maple Grove, MN 55311-1566 (US)
(72) Inventor: WHELEHAN, Jennifer, Westborough, MA 01581 (US); LYDECKER, Lauren, S., Millbury, MA 01527 (US); FREDRICKSON, Gerald, Westford, MA 01886 (US)
(74) Representative: Vossius & Partner Patentanwälte Rechtsanwälte mbB
(86) International application number: PCT/US2022/070333
(87) International publication number: WO 2022/178476

(56) References cited:
- US-A1- 2012 065 674
- US-A1- 2020 038 005

## Description

### Technical Field

This disclosure relates generally to a medical device for tissue treatment. More particularly, at least some embodiments of the disclosure relate to devices for treating tissue with an adhesive or an agent, via a patch.

### Background

The gastrointestinal (GI) tract may require treatment for various reasons including perforations, bleeding defects, post-surgical leaks, or other wounds of the tract. However, limited treatment options exist for managing such wounds. Options include surgical re-operation, which is relatively invasive and may have high morbidity and mortality rates. Thus, endoscopic delivery of a treatment aid may be a less invasive and more effective option, promoting natural healing of tissue.

US 2020/038005 A1 discloses systems and methods of delivering a patch to a target site of a patient. The patch may comprise a biomaterial such as chitosan or extracellular matrix and may be biocompatible and/or bioresorbable. The system may include an endoscope, a patch, and one of an instrument or a cap, the patch being coupled to the respective instrument or cap and detachable therefrom. Methods of delivering the patch to the target site may include introducing an endoscope into a gastrointestinal tract of a patient, e.g., the patch being in a folded or crimped configuration, navigating a distal end of the endoscope proximate a target site; and applying the patch to the target site while releasing the patch from the endoscope.

US 2012/065674 A1 discloses methods and materials for closing an opening (e.g., an incision) within a mammal. For example, methods and materials for closing a transluminal incision created during a natural orifice transluminal endoscopic surgery are shown.

### Summary of the Disclosure

The presently claimed invention provides a medical device according to claim 1. Further developments of the herein claimed invention are described in the dependent claims.

According to an example, a medical device may comprise a first tube, a second tube configured to sheath at least a portion of the first tube and configured to translate along a length of the first tube, an expandable device at a distal portion of the first tube, the expandable device having a collapsed state and an expanded state, a patch surrounding at least a portion of an outer surface of the expandable device, and a connector for holding the patch to the expandable device when the second tube covers the patch and the expandable device and for releasing the patch from the expandable device after the second tube uncovers the patch and the expandable device.

In another example, the first tube may include a lumen in fluid communication with the expandable device to supply a fluid to the expandable device. The connector may extend longitudinally along a portion of the expandable device. The connector may comprise a folded sheet forming a first half of the folded sheet and a second half of the folded sheet, wherein the first half of the folded sheet is attached to the expandable device, and the second half of the folded sheet is attached to the patch. The second half of the folded sheet may include a first surface adjacent to the first half of the folded sheet and a second surface adjacent to the patch, wherein the second surface is attached to the patch. The first half of the folded sheet may be attached to the expandable device via an adhesive, and the second half of the folded sheet may be attached to the patch via a soluble adhesive. The soluble adhesive may be configured to dissolve when the second tube uncovers the patch and the expandable device, and the patch may be exposed to a solvent, thereby releasing the patch from the connector and the expandable device.

In another example, the medical device may further comprise a wire, wherein a distal end of the wire is coupled to a distal portion of the first tube, the wire is sutured through the patch and the connector, and the wire extends proximally through a lumen of one of the first tube and the second tube. The wire may be sutured linearly through the patch and the connector, along a longitudinal length of the connector. The connector may include a plurality of openings configured to receive the sutured wire. A proximal end of the wire may be configured to be pulled so that the wire translates proximally, thereby removing the wire from the patch and the connector. The distal end of the wire may be removably secured to the distal portion of the first tube via a heat shrink or a crimp. The proximal end of the wire is coupled to an actuator configured to pull the wire proximally.

In another example, the connector may comprise a soluble adhesive directly between the patch and the expandable device, and the soluble adhesive is configured to dissolve when the second tube uncovers the patch and the expandable device and the soluble adhesive is exposed to a solvent, thereby releasing the patch from the expandable device. The patch may comprise chitosan and/or a chitosan modified material.

According to another example, a medical device may comprise a tube, an expandable device at a distal portion of the tube, the expandable device having a collapsed state and an expanded state, a patch surrounding at least a portion of an outer surface of the expandable device, the patch being removably attached to the expandable device via a connector.. The medical device may further comprise a wire suturing a portion of the connector to the patch, wherein the wire extends proximally along a length of the tube, and a proximal portion of the wire may be coupled to an actuator. The actuator may comprise a knob or a switch configured to pull the wire proximally, thereby removing the wire from the patch and the second half of the connector. The actuator may be positioned on a proximal portion of the tube.

According to an example, a method of treating a subject may comprise introducing a medical device into a gastrointestinal system of the subject, positioning a distal end of the medical device within a body lumen of the subject, wherein the distal end of the medical device includes an expandable device, a patch removably coupled to an outer surface of the expandable device, and a connector for holding the patch to the expandable device, retracting an outer sheath to expose the expandable device and the patch, supplying a fluid to the expandable device, such that the expandable device expands and the patch contacts a tissue surface of the bodily lumen, and releasing the patch from the expandable device.

### Brief Description Of The Drawings

The accompanying drawings, which are incorporated in and constitute a part of this specification, illustrate various exemplary embodiments and together with the description, serve to explain the principles of the disclosed embodiments.
FIGS. 1A-1B are side views of a portion of a medical device, according to an embodiment.
FIGS. 1C-1D are perspective views of a delivery feature of the medical device of FIGS. 1A-1B.
FIGS. 2A-2B are perspective views of a delivery feature, according to another embodiment.
FIG. 3 is a perspective view of a delivery feature, according to another embodiment.
FIGS. 4A-4B are side views of a portion of a medical device, according to an embodiment.

### Detailed Description

Reference will now be made in detail to aspects of the disclosure, examples of which are illustrated in the accompanying drawings. Wherever possible, the same or similar reference numbers will be used through the drawings to refer to the same or like parts. The term "distal" refers to a location or portion of a medical device farthest away from a user of the device, e.g., when introducing a device into a subject (e.g., patient). By contrast, the term "proximal" refers to a location or portion closest to the user, e.g., when placing the device into the subject.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus. In this disclosure, relative terms, such as, for example, "about," "substantially," "generally," and "approximately" are used to indicate a possible variation of ±10% in a stated value or characteristic.

Embodiments of this disclosure includes devices, systems, and methods for treating bodily wounds, e.g., endoluminal wounds, including the endoscopic delivery of a treatment aid. Examples of a treatment aid may include an adhesive or any suitable therapeutic agent, or a combination with an adhesive and a therapeutic agent. Said adhesive may be in the form of a patch, and said agent, in any suitable form, may be delivered via a patch. For example, the patch may be loaded with a therapeutic agent, and the patch may deliver the agent over time when in contact with tissue. Therefore, the patch may be configured to adhere to tissue and serve as a protective layer, and/or deliver agent to any bodily lumen, cavity, etc., for example, the GI tract.

The patch is not particularly limited. The patch may be a biodegradable and/or biocompatible patch of any suitable shape, e.g., a square, rectangle, etc., or any suitable dimension, e.g., 1 cm x 1 cm, depending on the treatment site. Moreover, the patch may be of any suitable material, e.g., polysaccharides (chitosan, cellulose, starch, alginates, etc.) that may be further modified with synthetic biocompatible materials (PGA, PLA, PCA, PEG, etc.) In some embodiments, the patch may be multi-layered, with each layer having different properties and characteristics to delay degradation over time. For example, each layer of a patch configured for agent delivery may comprise of a different ratio of natural/synthetic polymers to yield a timed release of the agent for an extended period of time. Thus, layers may degrade and the patch may continue to provide therapy.

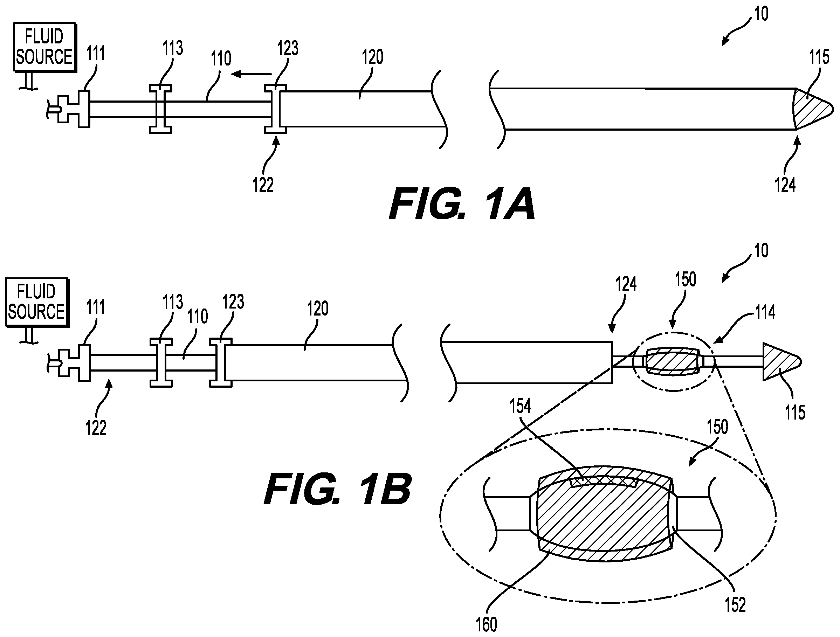

The patch may be delivered via a catheter, scope (endoscope, bronchoscope, colonoscope, etc.), tube, or sheath, inserted into the GI tract via a natural orifice. The orifice can be, for example, the nose, mouth, or anus, and the placement can be in any portion of the GI tract, including the esophagus, stomach, duodenum, large intestine, or small intestine. FIGS. 1A- 4B, further discussed below, illustrate embodiments of medical devices configured to deliver the patch to a lumen of the GI tract. It is noted that any one of these medical devices may be used with any suitable endoscope. For example, an endoscope may include a handle including a port, and the port may receive any one of the medical devices of FIGS. 1A-4B, which may extend distally via a working channel of the endoscope's shaft.

Referring to FIGS. 1A-1B, a medical device 10 includes an inner catheter 110, an outer sheath 120, and a delivery feature 150. Inner catheter 110 may be any suitable, biocompatible catheter including at least one inner channel (not shown), as well as at least one opening (not shown) further discussed below. Inner catheter 110 includes a proximal end including a luer 111, a distal end including a nose 115, and a handle 113. Luer 111 is not particularly limited, and may be any suitable luer configured to couple the proximal end of catheter 110 with a fluid source, so that the inner channel of catheter 110 may be in communication with said fluid source. The type of fluid is not particularly limited, and may be any suitable fluid, e.g., pressurized gas, saline. Nose 115 defines an atraumatic distal end of device 10, and is conical in shape, but not limited thereto. Handle 113 is not particularly limited, and may be of any suitable shape or size that may be gripped by a user of device 10. Handle 113 may be fixed to the outer surface of inner catheter 110, between luer 111 and a proximal end 122 of outer sheath 120.

Outer sheath 120 may be any suitable, biocompatible catheter configured to sheath a portion of inner catheter 110, and in some instances, delivery feature 150 (further discussed below). Outer sheath 120 includes proximal end 122 including a handle 123. Handle 123 is not particularly limited, and may be any suitable handle that is fixed to the outer surface of proximal end 122 or a proximal portion of sheath 120. A user may grip handle 123 to retract outer sheath 120 proximally, relative to inner catheter 110, thereby exposing delivery feature 150 to an external environment, e.g., a bodily lumen. Outer sheath 120 also includes a distal end 124, which may abut nose 115 when outer sheath 120 is translated distally, relative to inner catheter 110.

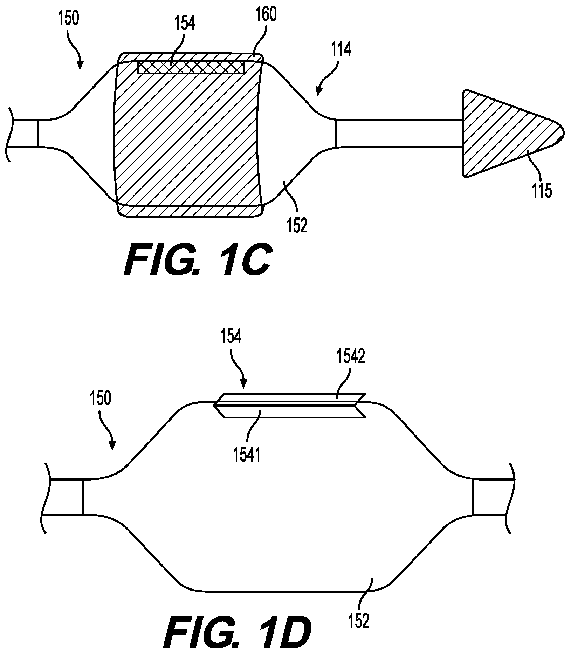

Referring to FIGS. 1B-1D, delivery feature 150 includes an expandable device, e.g., a balloon 152, a patch 160, and a connector 154. Delivery feature 150 may include a number of states: 1) a pre-deployment state, in which balloon 152 is in a deflated state (shown in FIG. 1B); 2) a deployment state, in which balloon 152 is in an inflated state (shown in FIG. 1C); 3) a post-deployment state, in which patch 160 is released from balloon 152, and balloon 152 is ready to return to a deflated state (shown in FIG. 1D); and 4) a withdrawal state (not shown), in which balloon 152 has been deflated, and device 10 may be withdrawn from the patient.

Balloon 152 may be an inflatable/deflatable feature of any suitable material, e.g., polymer, plastic, etc. The size and shape of balloon 152 is not particularly limited, and may be such that balloon 152, in an inflated state, contacts or abuts the targeted bodily lumen, e.g., the esophagus, colon, etc. For example, balloon 152 may be 1.5 cm to 2 cm in diameter when inflated, similar to the dimensions of a typical human esophagus. Balloon 152 maybe a compliant balloon (inflated by volume rather than pressure), non-compliant, or semi-compliant.

Balloon 152 may be fitted over a distal portion 114 of inner catheter 110, which may include at least one opening (not shown). Said at least one opening is in fluid communication with the inner channel of catheter 110, which in turn is in communication with the fluid source. Thus, said at least one opening may serve as an outlet by which balloon 152 may be inflated with fluid or deflated. The number of openings is not particularly limited. In a deflated state (shown in FIG. 1B), balloon 152 may be tightly raveled or wound about distal portion 114, thereby minimizing its radial profile relative to inner catheter 110. In an inflated state (shown in FIG. 1C), balloon 152 may unravel and expand to a size that allows the balloon (and patch 160) to contact the surrounding bodily lumen.

In some examples, a proximal end of balloon 152 or another aspect of inner catheter 110 may further include a pressure relief valve (not shown). Said pressure relief valve may be pre-set with a cut off pressure value, e.g., 4 psi (27579 pascal), thereby inhibiting over-inflation of balloon 152.
This helps to ensure that the body lumen is not perforated during the procedure. Device 10 may also include an additional vent, valve, or other structure by which fluid from balloon 152 may be released, thereby deflating balloon 152.

Patch 160, as discussed above, is not particularly limited. Patch 160 may be configured to adhere to tissue when in contact, and may also deliver agent to a bodily lumen of the GI tract. Patch 160 may be coupled to balloon 152, via connector 154 further discussed below. In the pre-deployment state (FIG. 1B), patch 160 may be tightly wound and spiraled around balloon 152, thereby minimizing the radial profile of delivery feature 150. When balloon 152 is inflated to transition feature 150 into a deployment state (FIG. 1C), patch 160 may simultaneously unravel and expand with balloon 152, so that patch 160 may contact and adhere to surrounding tissue. Patch 160 may cover all or only a circumferential portion of balloon 152 for treating less than a full circumference of a bodily lumen.

Connector 154 may be a sheet or an adhesive of any suitable material, e.g., paper, silicone, etc. According to the invention, connector 154 is folded into two halves - a first portion 1541 and a second portion 1542. First portion 1541 is adhered to an outer surface of balloon 152, via any suitable adhesive means, e.g., soluble, insoluble adhesives. For example, said adhesive means may be an insoluble adhesive so that first portion 1541 remains adhered to the outer surface of balloon 152. In another example, said adhesive means may be a soluble adhesive so that connector 154 may detach from balloon 152 after a duration of time. A surface of second portion 1542, that is adjacent to a surrounding patch 160 while connector 154 remains folded, may be attached to patch 160. Thus, connector 154 may serve as an intermediary connector between balloon 152 and patch 160. Second portion 1542 may be attached to patch 160 by any suitable dissolvable adhesive. For example, said adhesive may be a water-soluble adhesive, e.g., polyvinyl alcohol, etc. Thus, after inflation of balloon 152 and expansion of patch 160, patch 160 may be released from connector 154, via the dissolution of the adhesive between second portion 1542 and patch 160 (shown in FIG. 1D). It is noted that connector 154 is not limited to the example discussed above. In some instances, connector 154 may be of a single adhesive or sheet, with one side adhered to balloon 152 and the other adhered to patch 160.

Referring to FIGS. 1A-1D, an example of a method for delivery of patch 160 is further discussed. A user may introduce medical device 10 into a subject while delivery feature 150 is in a pre-deployment state, and outer sheath 120 is sheathing feature 150. For example, medical device 10 may be introduced into the body of a subject via a natural orifice such as a mouth or anus. Medical device 10 may traverse through a tortuous natural body lumen of the subject, such as an esophagus, stomach, colon, etc. Medical device 10 may be delivered via a scope or any other suitable way. A user may direct/position the distal end of medical device 10 within a targeted bodily lumen, e.g., esophagus, colon, etc. A user may then translate outer sheath 120 proximally by pulling on handle 123, thereby exposing delivery feature 150 to the lumen. A user may then supply fluid through inner catheter 110, e.g., by turning on the fluid source, to supply fluid to balloon 152, thereby inflating balloon 152 and transitioning feature 150 to a deployment state. Fluid may be supplied, for example, until balloon 152 and patch 160 contact the surrounding lumen. In some instances, this volume of fluid supplied may be determined according to the size of the lumen, or may be preset as a cutoff value for an associated pressure relief valve. Once patch 160 contacts and adheres to the surrounding lumen, patch 160 may be released naturally from connector 154 and balloon 152 via the dissolution of the dissolvable adhesive between connector 154 and patch 160. The user may then deflate balloon 152 by any suitable means, and retract device 10 proximally until device 10 is removed from the subject.

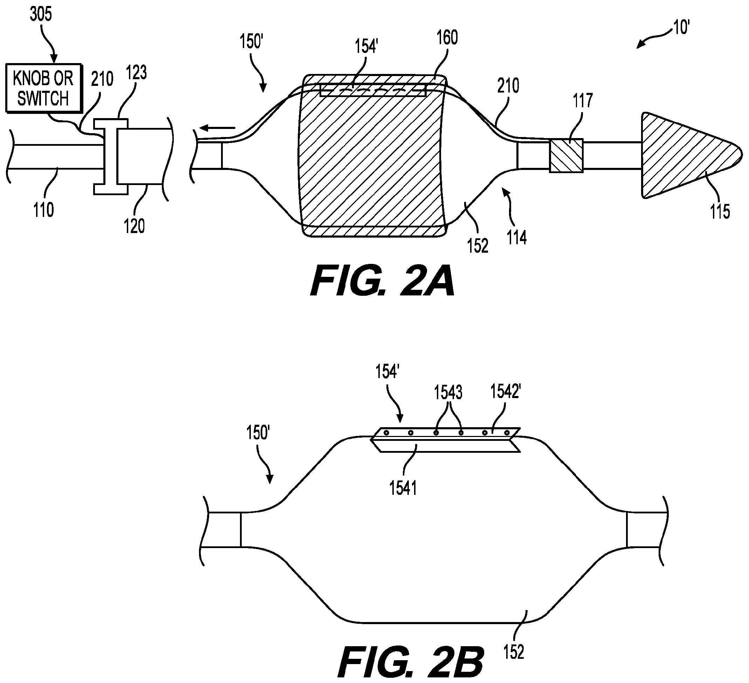

Referring to FIGS. 2A-2B, another example of a delivery feature 150' of device 10' is shown. In this example, like reference numerals refer to similar features described above in connection to delivery feature 150. Delivery feature 150' includes connector 154' and a wire 210. Connector 154' is similar to connector 154 of device 10 in many respects. However, second portion 1542' includes a plurality of openings 1543, arranged throughout a length of second portion 1542'. As shown in FIG. 2A, openings 1543 may be linearly arranged. However, in other examples, openings 1543 may be throughout connector 154' in any suitable arrangement. Openings 1543 may be of a sufficient width to receive wire 210, so that patch 160 may be sutured onto second portion 1542' via wire 210, as shown in FIG. 2A. Thus, patch 160 may be coupled to second portion 1542', without the use of an adhesive.

Wire 210 is not particularly limited, and may be any suitable wire-like feature, including suture material. Wire 210 may be of a length extending from a distal portion of device 10' to a point that is proximal to proximal end 122 of outer sheath 120. For example, a distal end of wire 210 may be secured under a heat shrink tube 117, or any other suitable mechanism, e.g., a crimp, which may be distal to feature 150' and fixed to catheter 110. Heat shrink tube 117 is not particularly limited. A portion of wire 210 proximal to its distal end may be sewn through patch 160 and second portion 1542', coupling second portion 1542' to patch 160. The remaining proximal portion of wire 210 may extend throughout the lumen of sheath 120, and past proximal end 122 of sheath 120. A proximal end of wire 210 may be secured or attached to a knob or switch 305. Knob or switch 305 may be positioned on any portion of device 10', e.g., handle 113, proximal to sheath 120, or may be separate from device 10'. Knob or switch 305 may be configured to wind or pull wire 210 proximally, so that the distal end of wire 210 may be released from heat shrink 117 tube and removed from patch 160 and second portion 1542', thereby releasing patch 160 from connector 154'. Alternatively, wire 210 may hang out of proximal end 122 of sheath 120 for a user to manually pull, without any intervening mechanical features. In other examples, delivery feature 150' may include a plurality of wires 210, sewn through patch 160 and second portion 1542'. The additional wires may further secure patch 160 onto second portion 1542'.

Medical device 10' may be used in a similar manner as medical device 10, except a user may pull wire 210 proximally to deploy patch 160 from connector 154' and balloon 152. Wire 210 may be pulled by any suitable means. In some examples, a user many actuate knob or switch 305 which winds wire 210 proximally, so that wire 210 may be removed from connector 154'. In other examples, a user may manually pull on wire 210 to do the same.

It is noted suturing is not limited to coupling patch 160 onto connector 154'. In other examples, additional aspects of the medical device 10' may be sutured together as well. For example, delivery feature 150', in a pre-deployment state, may be packaged or contained in a wrapper of any suitable, biocompatible material. Said wrapper may be sutured onto an outer surface of patch 160, via a second wire. Thus, in addition to wire 210, a second wire may extend proximally along the length of device 10'. In such an example, device 10' may be without outer sheath 120, as delivery feature 150' may be contained within the sutured wrapper. To expose delivery feature 150', the second wire may be pulled proximally to remove the second wire, and release said wrapper from patch 160.

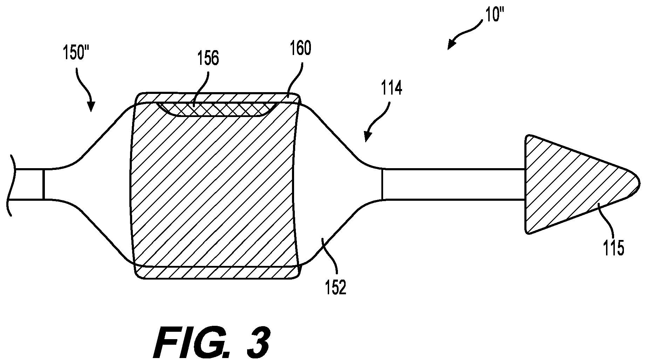

Referring to FIG. 3, another example of a delivery feature 150" of device 10" is shown. In this example, like reference numerals refer to similar features described above in connection to delivery features 150, 150'. Balloon 152 may be coupled to patch 160, via a soluble adhesive 156, e.g., polyvinyl alcohol. Adhesive 156 may be on all or a portion of an inner surface of patch 160, or on all or a portion of an outer surface of balloon 152. Thus, when delivery feature 150" is exposed and in a deployment state, patch 160 may be released from balloon 152 as adhesive 156 dissolves. Thus, medical device 10" may be used in the same manner as device 10, as discussed above.

It is noted connectors 154, 154' and adhesive 156, discussed above, extend along only a portion of balloon 152. However, delivery features 150, 150' 150" are not limited to a single connector 154, 154' or a single spot of adhesive 156. In some examples, said features may have a plurality of their respective connectors 154, 154' or adhesive 156 at different radial and longitudinal positions throughout balloon 152, for greater adherence with patch 160 to balloon 152.

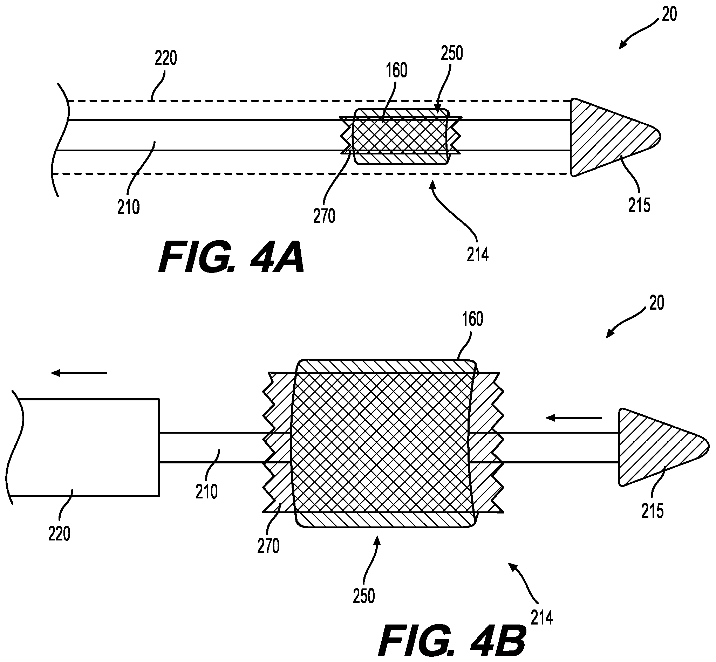

FIGS. 4A-4B show another exemplary medical device 20, which may include any of the features of medical device 10, 10', 10" discussed above. Medical device 20 includes an inner catheter 210 (which may be the same or similar to catheter 110), an outer sheath 220 (which may be the same or similar to sheath 120), a nose 215 (which may be the same or similar to nose 115), and a delivery feature 250. Delivery feature 250 includes a stent 270 and patch 160.

Stent 270 is not particularly limited, and may be any suitable, bio-absorbable stent. Stent 270 may be of any flexible and elastic-like material, e.g., PLLA, so that stent 270 may expand to its default shape after being adjusted or manipulated. Stent 270 may be of a shape memory material. The size and shape of stent 270 is not limited, and may depend on the dimensions of the bodily lumen, e.g., esophagus, colon, etc., to be treated. For example, stent 270 may be of a sufficient size to apply a necessary degree of pressure against the surrounding walls of a bodily lumen, without causing a perforation. This may also reduce the likelihood of undesired migration of stent 270. Furthermore, stent 270, in an expanded, default state, may be of a sufficient width to enable the proximal retraction of inner catheter 210 and nose 215 through a lumen of stent 270. As discussed above, patch 160 is not particularly limited, and may be wrapped or folded around stent 270 via any suitable manner. In some instances, patch 160 may be coupled to stent 270 in a manner that would allow for patch 160 to expand as stent 270 expands. It is noted that patch 160 may be of a sufficient size to fully cover stent 270 in its expanded, original state. This may also reduce risk of ripping patch 160 upon expansion of stent 270. However, in some examples, patch 160 may cover only a circumferential portion of stent 270 for treating less than a full circumference of a bodily lumen.

Delivery feature 250 includes a pre-deployment state (shown in FIG. 4A) and a deployment state (shown in FIG. 4B). In a pre-deployment state, delivery feature 250 is sheathed by outer sheath 220, and stent 270 is collapsed or compressed about a distal portion 214 of inner catheter 210. Stent 270 may be compressed and patch 160 may be wrapped and folded around stent 270 to minimize the radial profile of feature 250, thereby allowing feature 250 to be sheathed by outer sheath 220. In some examples, patch 160 may be affixed to an axial portion of stent 270, and wound around stent 270 until deployment. The manner in which patch 160 may be affixed is not particularly limited, and may be, for example, via a biodegradable adhesive, or a heat or solvent weld. In some other examples, patch 160 may be attached to the distal or proximal end of stent 270 at particular points around the circumference, where patch 160 is then folded and pleated, and wrapped around stent 270 as it is pulled taught to be loaded into outer sheath 220. While sheathed, stent 270 may remain in a compressed state, due to the surrounding inner surfaces of sheath 220. In a deployment state, delivery feature 250 may be exposed to a surrounding lumen when sheath 220 is translated proximally relative to catheter 210. With the removal of any surrounding surfaces, stent 270, given its flexible, elastic characteristic, may expand to its original shape. As stent 270 expands, patch 160 may also unfold simultaneously, while still covering stent 270. It is noted that to maintain patch 160 on stent 270, any of the connectors 154, 154' (and associated adhesive, wire 210, etc.) may be used in delivery feature 250. Stent 270 may degrade over time in the bodily lumen.

Medical device 20 may be used in a similar manner as medical device 10, except there is no inflation or deflation of an expandable device, e.g., a balloon. Rather, a user may translate outer sheath 220 proximally via any suitable manner, thereby exposing delivery feature 250 to the surrounding lumen. Subsequently thereafter, stent 270 will expand towards its original size and shape, and patch 160 will unfold and expand simultaneously. Once stent 270 (and surrounding patch 160) is deployed and stabilized within the targeted lumen, a user may then retract device 20 proximally until device 20 is removed from the subject.

## Claims

1. A medical device (10, 10', 10", 20), comprising:
a first tube (110);
a second tube (120) configured to sheath at least a portion of the first tube and configured to translate along a length of the first tube;
an expandable device (152) at a distal portion (114, 214) of the first tube, the expandable device having a collapsed state and an expanded state;
a patch (160) surrounding at least a portion of an outer surface of the expandable device; and
a connector (154, 154') for holding the patch to the expandable device when the second tube covers the patch and the expandable device and for releasing the patch from the expandable device after the second tube uncovers the patch and the expandable device,
**characterized in that**
the connector comprises a folded sheet forming a first half (1541) and a second half (1542) of the folded sheet, wherein the first half of the folded sheet is attached to the expandable device, and the second half of the folded sheet is attached to the patch.

2. The medical device (10, 10', 10", 20) of claim 1, wherein the first tube (110) includes a lumen in fluid communication with the expandable device (152) to supply a fluid to the expandable device.

3. The medical device (10, 10', 10", 20) of claims 1-2, wherein the connector (154, 154') extends longitudinally along a portion of the expandable device (152).

4. The medical device (10, 10', 10", 20) of any one of claims 1 to 3, wherein the second half of the folded sheet includes a first surface adjacent to the first half of the folded sheet and a second surface adjacent to the patch (160), wherein the second surface is attached to the patch.

5. The medical device (10, 10', 10", 20) of any one of claims 1 to 4, wherein the first half of the folded sheet is attached to the expandable device (152) via an adhesive (156), and the second half of the folded sheet is attached to the patch (160) via a soluble adhesive (156).

6. The medical device (10, 10', 10", 20) of claim 5, wherein the soluble adhesive (156) is configured to dissolve when the second tube (120) uncovers the patch (160) and the expandable device (152), and the patch is exposed to a solvent, thereby releasing the patch from the connector (154, 154') and the expandable device.

7. The medical device (10, 10', 10", 20) of any of the preceding claims, further comprising a wire (210),
wherein a distal end of the wire is coupled to a distal portion (114, 214) of the first tube (110), the wire is sutured through the patch (160) and the connector (154, 154'), and the wire extends proximally through a lumen of one of the first tube and the second tube (120).

8. The medical device (10, 10', 10", 20) of claim 7, wherein the wire (210) is sutured linearly through the patch (160) and the connector (154, 154'), along a longitudinal length of the connector.

9. The medical device (10, 10', 10", 20) of claim 7 or 8, wherein the connector (154, 154') includes a plurality of openings (1543) configured to receive the sutured wire.

10. The medical device (10, 10', 10", 20) of any of claims 7 to 9, wherein a proximal end of the wire (210) is configured to be pulled so that the wire translates proximally, thereby removing the wire from the patch (160) and the connector (154, 154').

11. The medical device (10, 10', 10", 20) of claim 10, wherein the distal end of the wire (210) is removably secured to the distal portion (114, 214) of the first tube (110) via a heat shrink (117) or a crimp.

12. The medical device (10, 10', 10", 20) of claim 10 or 11, wherein the proximal end of the wire (210) is coupled to an actuator configured to pull the wire proximally.

13. The medical device (10, 10', 10", 20) of any of claims 1-3, wherein the connector (154, 154') comprises a soluble adhesive (156) directly between the patch (160) and the expandable device (152), and the soluble adhesive is configured to dissolve when the second tube (120) uncovers the patch and the expandable device and the soluble adhesive is exposed to a solvent, thereby releasing the patch from the expandable device.

14. The medical device (10, 10', 10", 20) of any of the preceding claims, wherein the patch (160) comprises chitosan and/or a chitosan modified material.

## Patentansprüche

1. Medizinische Vorrichtung (10, 10', 10"; 20), aufweisend:
einen ersten Schlauch (110);
einen zweiten Schlauch (120), der dazu konfiguriert ist, mindestens einen Abschnitt des ersten Schlauchs zu umhüllen und sich entlang einer Länge des ersten Schlauchs zu verschieben; eine expandierbare Vorrichtung (152) an einem distalen Abschnitt (114, 214) des ersten Schlauchs, wobei die expandierbare Vorrichtung einen kollabierten Zustand und einen expandierten Zustand aufweist;
ein Pflaster (160), das mindestens einen Abschnitt einer Außenfläche der expandierbaren Vorrichtung umgibt;
und
ein Verbindungsstück (154, 154') zum Halten des Pflasters an der expandierbaren Vorrichtung, wenn der zweite Schlauch das Pflaster und die expandierbare Vorrichtung bedeckt, und zum Lösen des Pflasters von der expandierbaren Vorrichtung, nachdem der zweite Schlauch das Pflaster und die expandierbare Vorrichtung freilegt,
**dadurch gekennzeichnet, dass**
das Verbindungsstück ein gefaltetes Blatt aufweist, das eine erste Hälfte (1541) und eine zweite Hälfte (1542) des gefalteten Blattes bildet, wobei die erste Hälfte des gefalteten Blattes an der expandierbaren Vorrichtung angebracht ist und die zweite Hälfte des gefalteten Blattes an dem Pflaster angebracht ist.

2. Medizinische Vorrichtung (10, 10', 10", 20) nach Anspruch 1, wobei der erste Schlauch (110) ein Lumen in Fluidverbindung mit der expandierbaren Vorrichtung (152) aufweist, um der expandierbaren Vorrichtung ein Fluid zuzuführen.

3. Medizinische Vorrichtung (10, 10', 10", 20) nach Anspruch 1-2, wobei sich das Verbindungsstück (154, 154') in Längsrichtung entlang eines Abschnitts der expandierbaren Vorrichtung (152) erstreckt.

4. Medizinische Vorrichtung (10, 10', 10", 20) nach einem der Ansprüche 1 bis 3, wobei die zweite Hälfte des gefalteten Blattes eine erste Fläche angrenzend an die erste Hälfte des gefalteten Blattes und eine zweite Fläche angrenzend an das Pflaster (160) aufweist, wobei die zweite Fläche an dem Pflaster angebracht ist.

5. Medizinische Vorrichtung (10, 10', 10", 20) nach einem der Ansprüche 1 bis 4, wobei die erste Hälfte des gefalteten Blattes über einen Klebstoff (156) an der dehnbaren Vorrichtung (152) und die zweite Hälfte des gefalteten Blattes über einen löslichen Klebstoff (156) an dem Pflaster (160) befestigt ist.

6. Medizinische Vorrichtung (10, 10', 10", 20) nach Anspruch 5, wobei der lösliche Klebstoff (156) dazu konfiguriert ist, sich aufzulösen, wenn der zweite Schlauch (120) das Pflaster (160) und die expandierbare Vorrichtung (152) freilegt und das Pflaster einem Lösungsmittel ausgesetzt wird, wodurch das Pflaster von dem Verbindungsstück (154, 154') und der expandierbaren Vorrichtung gelöst wird.

7. Medizinische Vorrichtung (10, 10', 10", 20) nach einem der vorstehenden Ansprüche, ferner aufweisend einen Draht (210),
wobei ein distales Ende des Drahts mit einem distalen Abschnitt (114, 214) des ersten Schlauchs (110) gekoppelt ist, der Draht das Pflaster (160) und das Verbindungsstück (154, 154') vernäht und sich der Draht proximal durch ein Lumen des ersten Schlauchs oder des zweiten Schlauchs (120) erstreckt.

8. Medizinische Vorrichtung (10, 10', 10", 20) nach Anspruch 7, wobei der Draht (210) das Pflaster (160) und das Verbindungsstück (154, 154') entlang einer Längserstreckung des Verbindungsstücks linear vernäht.

9. Medizinische Vorrichtung (10, 10', 10", 20) nach Anspruch 7 oder 8, wobei das Verbindungsstück (154, 154') mehrere Öffnungen (1543) aufweist, die zum Aufnehmen des vernähten Drahts konfiguriert sind.

10. Medizinische Vorrichtung (10, 10', 10", 20) nach einem der Ansprüche 7 bis 9, wobei ein proximales Ende des Drahts (210) dazu konfiguriert ist, derart gezogen zu werden, dass sich der Draht nach proximal verschiebt, wodurch der Draht aus dem Pflaster (160) und dem Verbindungsstück (154, 154') entfernt wird.

11. Medizinische Vorrichtung (10, 10', 10", 20) nach Anspruch 10, wobei das distale Ende des Drahts (210) über ein Warmschrumpfteil (117) oder eine Crimpung lösbar an dem distalen Abschnitt (114, 214) des ersten Schlauchs (110) befestigt ist.

12. Medizinische Vorrichtung (10, 10', 10", 20) nach Anspruch 10 oder 11, wobei das proximale Ende des Drahts (210) mit einem Aktuator gekoppelt ist, der dazu konfiguriert ist, den Draht nach proximal zu ziehen.

13. Medizinische Vorrichtung (10, 10', 10", 20) nach einem der Ansprüche 1-3, wobei das Verbindungsstück (154, 154') einen löslichen Klebstoff (156) direkt zwischen dem Pflaster (160) und der expandierbaren Vorrichtung (152) aufweist und der lösliche Klebstoff dazu konfiguriert ist, sich aufzulösen, wenn der zweite Schlauch (120) das Pflaster und die expandierbare Vorrichtung freilegt und der lösliche Klebstoff einem Lösungsmittel ausgesetzt wird, wodurch das Pflaster von der expandierbaren Vorrichtung gelöst wird.

14. Medizinische Vorrichtung (10, 10', 10", 20) nach einem der vorstehenden Ansprüche, wobei das Pflaster (160) Chitosan und/oder ein mit Chitosan modifiziertes Material aufweist.

## Revendications

1. Dispositif médical (10, 10', 10", 20), comprenant:
un premier tube (110);
un deuxième tube (120) configuré pour envelopper au moins une partie du premier tube et configuré pour se déplacer le long d'une longueur du premier tube;
un dispositif extensible (152) au niveau d'une partie distale (114, 214) du premier tube, le dispositif extensible ayant un état replié et un état étendu;
un patch (160) entourant au moins une partie d'une surface extérieure du dispositif extensible;
et
un connecteur (154, 154') pour maintenir le patch sur le dispositif extensible lorsque le deuxième tube recouvre le patch et le dispositif extensible et pour libérer le patch du dispositif extensible après que le deuxième tube découvre le patch et le dispositif extensible,
**caractérisé en ce que**
le connecteur comprend une feuille pliée formant une première moitié (1541) et une deuxième moitié (1542) de la feuille pliée, dans lequel la première moitié de la feuille pliée est fixée au dispositif extensible, et la deuxième moitié de la feuille pliée est fixée au patch.

2. Dispositif médical (10, 10', 10", 20) selon la revendication 1, dans lequel le premier tube (110) inclut une lumière en communication fluidique avec le dispositif extensible (152) pour fournir un fluide au dispositif extensible.

3. Dispositif médical (10, 10', 10", 20) selon les revendications 1 et 2, dans lequel le connecteur (154, 154') s'étend longitudinalement le long d'une partie du dispositif extensible (152).

4. Dispositif médical (10, 10', 10", 20) selon l'une quelconque des revendications 1 à 3, dans lequel la deuxième moitié de la feuille pliée inclut une première surface adjacente à la première moitié de la feuille pliée et une deuxième surface adjacente au patch (160), dans lequel la deuxième surface est fixée au patch.

5. Dispositif médical (10, 10', 10", 20) selon l'une quelconque des revendications 1 à 4, dans lequel la première moitié de la feuille pliée est fixée au dispositif extensible (152) par l'intermédiaire d'un adhésif (156), et la deuxième moitié de la feuille pliée est fixée au patch (160) par l'intermédiaire d'un adhésif soluble (156).

6. Dispositif médical (10, 10', 10", 20) selon la revendication 5, dans lequel l'adhésif soluble (156) est configuré pour se dissoudre lorsque le deuxième tube (120) découvre le patch (160) et le dispositif extensible (152), et que le patch est exposé à un solvant, libérant ainsi le patch du connecteur (154, 154') et du dispositif extensible.

7. Dispositif médical (10, 10', 10", 20) selon l'une quelconque des revendications précédentes, comprenant en outre un fil (210),
dans lequel une extrémité distale du fil est couplée à une partie distale (114, 214) du premier tube (110), le fil suturant le patch (160) et le connecteur (154, 154'), et le fil s'étend de manière proximale à travers une lumière de l'un du premier tube et du deuxième tube (120).

8. Dispositif médical (10, 10', 10", 20) selon la revendication 7, dans lequel le fil (210) suture linéairement le patch (160) et le connecteur (154, 154'), le long d'une longueur longitudinale du connecteur.

9. Dispositif médical (10, 10', 10", 20) selon la revendication 7 ou 8, dans lequel le connecteur (154, 154') inclut une pluralité d'ouvertures (1543) configurées pour recevoir le fil amené à suturer.

10. Dispositif médical (10, 10', 10", 20) selon l'une quelconque des revendications 7 à 9, dans lequel une extrémité proximale du fil (210) est configurée pour être tirée de sorte que le fil se déplace de manière proximale, retirant ainsi le fil du patch (160) et du connecteur (154, 154').

11. Dispositif médical (10, 10', 10", 20) selon la revendication 10, dans lequel l'extrémité distale du fil (210) est fixée de manière amovible à la partie distale (114, 214) du premier tube (110) par l'intermédiaire d'une gaine thermorétractable (117) ou d'un sertissage.

12. Dispositif médical (10, 10', 10", 20) selon la revendication 10 ou 11, dans lequel l'extrémité proximale du fil (210) est couplée à un organe d'actionnement configuré pour tirer le fil de manière proximale.

13. Dispositif médical (10, 10', 10", 20) selon l'une quelconque des revendications 1 à 3, dans lequel le connecteur (154, 154') comprend un adhésif soluble (156) directement entre le patch (160) et le dispositif extensible (152), et l'adhésif soluble est configuré pour se dissoudre lorsque le deuxième tube (120) découvre le patch et le dispositif extensible et que l'adhésif soluble est exposé à un solvant, libérant ainsi le patch du dispositif extensible.

14. Dispositif médical (10, 10', 10", 20) selon l'une quelconque des revendications précédentes, dans lequel le patch (160) comprend du chitosane et/ou un matériau modifié au chitosane.
